# EUROPÄISCHE PATENTSCHRIFT

(11) **EP 4 659 713 B1**
(45) Veröffentlichungstag und Bekanntmachung des Hinweises auf die Patenterteilung: **23.09.2026**
(21) Anmeldenummer: 25202371.8
(22) Anmeldetag: 28.03.2023
(51) Int. Cl.: A61F 2/46, A61F 2/30, A61F 2/36, A61F 2/40

(54) **VORRICHTUNG UND VERFAHREN ZUR HERSTELLUNG VON HÜFTSPACERN**
DEVICE AND METHOD FOR PRODUCING HIP SPACERS
APPAREIL ET PROCÉDÉ DE FABRICATION D'ESPACEURS DE HANCHE

(43) Veröffentlichungstag der Anmeldung: 10.12.2025
(62) Dokumentnummer(n) der früheren Anmeldung(en) nach Art. 76 EPÜ: 23164506.0 / 4 438 010
(73) Patentinhaber: Heraeus Medical GmbH, 61273 Wehrheim (DE)
(72) Erfinder: Schmitz, Michael, 63450 Hanau (DE); Grün, Harald, 54636 Messerich (DE); Kunkel, Christian, 97828 Marktheidenfeld (DE); Pohlmann, Jochen, 97828 Marktheidenfeld (DE)
(74) Vertreter: Heraeus IP

(56) Entgegenhaltungen:
- EP-A1- 2 522 310
- EP-B1- 2 787 928
- WO-A2-2009/073781
- CN-A- 102 579 166

## Beschreibung

### GEBIET DER ERFINDUNG

Die vorliegende Erfindung betrifft das Gebiet der Medizintechnik, insbesondere Vorrichtungen zur Herstellung medizinischer Implantate, und die damit herstellbaren medizinischen Implantate.

Die Erfindung betrifft insbesondere eine Vorrichtung zum Herstellen eines Spacers durch Aushärten von Knochenzementteig. Der Spacer ist als temporärer Platzhalter dazu vorgesehen, im medizinischen Anwendungsbereich ein Gelenk oder einen Teil eines Gelenks, welches eine artikulierende Oberfläche eines Gelenkkopfs aufweist, temporär zu ersetzen. Bevorzugt ist der Spacer zum temporären Ersetzen eines Hüftgelenks oder eines Schultergelenks geeignet und vorgesehen, besonders bevorzugt zum temporären Ersetzen eines Hüftgelenks.

### TECHNISCHER HINTERGRUND

Gelenk-Endoprothesen, wie Hüftgelenk-Endoprothesen und Schultergelenk-Endoprothesen, werden in großem Umfang weltweit implantiert. Leider kommt es in einem geringen Prozentsatz vor, dass Gelenk-Endoprothesen von mikrobiellen Keimen, besonders Gram-positiven Bakterien als auch Gram-negativen Bakterien und in sehr geringem Umfang von Hefen und Pilzen, besiedelt werden. Diese mikrobiellen Keime, hauptsächlich typische Hautkeime wie *Staphylococcus aureus* und *Staphylococcus epidermidis,* können während einer chirurgischen Operation (OP) in den Patienten gelangen. Daneben ist es auch möglich, dass mikrobielle Keime hämatogen Gelenk-Endoprothesen erreichen. Bei einer Besiedlung von Gelenk-Endoprothesen mit mikrobiellen Keimen wird das umliegende Knochen- und Weichgewebe mit infiziert und von den mikrobiellen Keimen geschädigt.

Es gibt zwei vorherrschende Behandlungsverfahren für infizierte Gelenk-Endoprothesen, die einzeitige septische Revision und die zweizeitige septische Revision. Bei der einzeitige Revision wird innerhalb einer OP zuerst die infizierte Gelenk-Endoprothesen entfernt, anschließend radikal debridiert und dann wird eine Revisions-Gelenk-Endoprothese implantiert. Bei zweizeitigen septischen Revisionen wird in einer ersten OP zuerst die infizierte Gelenk-Endoprothese entfernt, dann wird debridiert und anschließend erfolgt die Implantation eines Spacers. Ein Hüftgelenkspacer besteht aus einem Schaft, einem Kragen, einem Hals und einem Kugelkopf und ist den Hüftgelenk-Endoprothesen in Form und Größe nachgebildet. Analog ist ein Schultergelenkspacer einer Schultergelenk-Endoprothese in Form und Größe nachgebildet. Der Spacer wird mit Knochenzement an dem jeweiligen Knochen verankert, also beispielsweise bei Hüftgelenkspacern am proximalen Femur beziehungsweise im Femurkanal verankert. Der Spacer verbleibt üblicherweise bis zu mehrere Wochen im Patienten, bis die Entzündung abgeklungen ist und die klinischen Entzündungsmarker zurückgegangen sind. Dann wird in einer zweiten OP der Spacer entfernt und nach erneutem Debridement eine Revisions-Gelenk-Endoprothese implantiert.

Im Rahmen von zweizeitigen septischen Wechseloperationen von Hüft- und Schultertotalgelenkendoprothesen sind Spacer als temporäre Platzhalter in der Interimsphase von wesentlicher Bedeutung. Für die intraoperative Herstellung von Spacern mit Polymethylmethacrylat-(PMMA-)Knochenzement sind Gießformen aus Kunststoff bekannt. Beispiele für solche Gießformen sind beispielsweise in US6,361,731B1, US7,637,729B2, US7,789,646B2, US8,480,389B2, EP 3957280A1 und US8,801,983B2 beschrieben.

EP 2787928B1 wird als nächstliegender Stand der Technik betrachtet und offenbart den Gegenstand der Präambel des Anspruchs 1.

### AUSFÜHRLICHE BESCHREIBUNG

Die vorliegende Erfindung ist in Anspruch 1 definiert und stellt Gießformen bereit, welche zur Herstellung von Gelenkspacern aus Knochenzementteig dienen können.

Dementsprechend ist die Gießform bevorzugt zum Herstellen eines Hüftgelenkspacers oder eines Schultergelenkspacers vorgesehen. Die Erfindung betrifft auch ein Kit zur Herstellung eines solchen Spacers mit einer solchen Vorrichtung.

Gegenstand der Erfindung ist somit insbesondere eine Vorrichtung in Form einer mehrteiligen Gießform zur Herstellung von einteiligen Hüft- und Schulterspacern, wobei die Gießform ein Kopfelement und ein Stammelement aufweist, und ein Metallkern zum Aufbau des Spacers verwendet werden kann. Hüft- und Schulterspacer sind als temporäre Platzhalter (Spacer) im Rahmen von zweizeitigen Revisionen von infizierten Hüft- und Schultertotalgelenkendoprothesen für die Interimsphase bestimmt. Die Vorrichtung kann für die Herstellung von Hüft- und Schulterspacern mit niedrigviskosem und hochviskosem Polymethylmethacrylat-Knochenzementteig geeignet sein.

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist. Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einer Gießform beschrieben werden, auch für die hierin beschriebenen Kits, Spacer und Verfahren anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Ein Aspekt der Erfindung betrifft in einer ersten Ausführungsform eine Gießform zur Herstellung eines Spacers, beispielsweise eines Hüftgelenkspacers, aufweisend ein Kopfelement, welches eine Oberschale und eine Unterschale aufweist, und ein Stammelement, welches eine Oberschale und eine Unterschale aufweist, wobei die Gießform ein Rastelement aufweist, und eine Oberschale und eine Unterschale des Kopfelements und/oder des Stammelements mithilfe des Rastelements miteinander verbindbar sind.

Das Kopfelement kann ausgebildet und eingerichtet sein, einen Teil eines Spacers abzuformen, welcher in die Gelenkpfanne eines Hüftgelenks eingreifen kann.

Das Stammelement kann ausgebildet und eingerichtet sein, einen Teil eines Spacers abzuformen, welcher in einen Femurknochen oder Oberarmknochen implantierbar ist.

Die Gießform weist weiterhin ein Halselement auf, welches zwischen dem Kopfelement und dem Stammelement angeordnet ist.

Bevorzugt ist die Gießform dazu eingerichtet, derartige Halselemente mit variabler Länge aufzunehmen, wie nachfolgend ausführlicher beschrieben. Dies kann unter anderem durch die hierin beschriebenen Ausführungsformen einer modularen Bauweise einer Gießform erzielt werden, in welchen Kopfelemente, Stammelemente und Halselemente verschiedener Größe untereinander kombinierbar sind.

Die Kopfelemente, Stammelemente und Halselemente sind jeweils als getrennte, aber miteinander verbindbare Elemente ausgestaltet.

Die Gießform weist zusätzlich zu dem Rastelement ein formschlüssiges Verbindungselement auf, um die Oberschale und die Unterschale des Kopfelements und/oder des Stammelements miteinander zu verbinden. Ein solches Verbindungselement kann beispielsweise aus Stegen und Vertiefungen bestehen, die in geeigneter Weise gegenüberliegend auf Oberschale und Unterschale angeordnet sind, um ineinander eingreifen zu können. Solche Stege und Vertiefungen können orthogonale Kanten aufweisen, welche ineinandergreifen, oder abgerundete Formen wie zum Beispiel halbzylindrisch geformte Rillen oder Halbkugeln umfassen. Dieses Verbindungselement kann dazu dienen, Oberschale und Unterschale aneinander auszurichten und/oder aneinander zu befestigen. Das Verbindungselement kann die Funktion des Rastelements unterstützen, in dem es Oberschale und Unterschale zueinander in einer gewünschten Position hält.

Das Rastelement ist ein Verbindungselement, welches mehrere Teile der Gießform mithilfe einer formschlüssigen Verbindung miteinander verbinden kann. Das Rastelement kann in Form einer Lasche ausgebildet sein. Die Lasche kann eine zungenförmige, biegsame Struktur aufweisen. Die Lasche kann auf der Außenseite eine glatte Oberfläche besitzen. Es können mehrere gleichartige oder verschiedenartige Laschen vorgesehen sein. Das Rastelement kann an einer Oberschale oder an einer Unterschale des Kopfelements angeordnet sein. Das Rastelement kann einen einer Oberschale oder an eine Unterschale des Stammelements angeordnet sein. Das Rastelement kann an der Oberschale des Kopfelements angeordnet sein, und eingerichtet sein, in eine geeignete Struktur einzugreifen, welche an der Unterschale des Kopfelements angeordnet ist. Beispielsweise kann das Rastelement als laschenförmiger Schnapphaken auf einer Oberschale ausgestaltet sein, der angeordnet und eingerichtet ist, in einen Vorsprung auf einer Unterschale einzugreifen. Alternativ oder zusätzlich kann die Gießform ein Rastelement aufweisen, welches als laschenförmiger Schnapphaken auf einer Unterschale ausgestaltet ist, der angeordnet und eingerichtet ist, in einen Vorsprung auf einer Oberschale einzugreifen. Hier sind unterschiedliche Kombinationen möglich, zum Beispiel kann die Oberschale des Kopfelements sowohl mehrere gleichartige als auch mehrere unterschiedliche Rastelemente aufweisen, während die Oberschale des Stammelements lediglich mehrere gleichartige Rastelemente aufweist.

Die verschiedenartigen Rastelemente können sich beispielsweise voneinander dadurch unterscheiden, dass sie trotz einer grundsätzlich gleichen Grundstruktur, zum Beispiel ein laschenförmiger Schnapphaken, ein zusätzliches Verbindungselement aufweisen, zum Beispiel eine Spundung nach dem Nut-/Feder-Prinzip.

Das Kopfelement und/oder das Stammelement können allgemein mehrere gleichartige Rastelemente aufweisen. Es können auch jeweils mehrere unterschiedliche Rastelemente an dem Kopfelement und/oder dem Stammelement vorgesehen sein.

In einer Ausführungsform ist das Rastelement angeordnet und eingerichtet, in einem vollständig zusammengefügten Zustand der Gießform einen Hohlraum zur Aufnahme von überschüssigem Knochenzementteig zu definieren. Obwohl in bevorzugten Ausführungsformen ein Austritt vor allem über dafür vorgesehene Entlüftungsöffnungen vorgesehen ist, kann es beispielsweise durch Fehlbedienung des Anwenders auch an der Verbindungskante zwischen Oberschale und Unterschale zu Austritt von Knochenzementteig kommen. In der hier beschriebenen Ausführungsform ist diese Austrittsstelle durch das Rastelement bedeckt, wobei das Rastelement beispielsweise als Lasche ausgestaltet sein kann. Der austretende Knochenzementteig kann dann in einem Hohlraum aufgenommen werden, welcher sich zwischen dem Rastelement und der Außenwandung der Gießform befindet.

Die Gießform weist weiterhin ein Halselement auf, welches mit dem Kopfelement und dem Stammelement verbindbar ist. Das Halselement kann eine ringförmige Grundform aufweisen.

In einer Ausführungsform weist das Halselement weiterhin einen Steg auf.

Der Steg kann parallel zur Mittelachse des Halselements angeordnet sein. Der Steg kann angeordnet und eingerichtet sein, in eine zu dem Steg komplementäre Struktur einzugreifen, die an dem Kopfelement und/oder dem Stammelement angeordnet ist. Hierdurch kann das Halselement mit dem Kopfelement bzw. dem Stammelement verbunden werden und im Verhältnis zu diesem in einer gewünschten Position gehalten werden.

In einer Ausführungsform weist das Halselement einen Schlitz auf. Der Schlitz kann als Unterbrechung der Mantelfläche des Halselements ausgeführt sein. Der Schlitz kann parallel zur Mittelachse des Halselements angeordnet sein. Der Schlitz kann dazu eingerichtet sein, ein Auseinanderbiegen oder Zusammendrücken des Halselements zu ermöglichen. Hierdurch kann das Halselement leichter von einem mithilfe der Gießform hergestellten Spacer entfernt werden, d. h. das Auseinanderbauen der Gießform zur Entnahme des Spacers wird durch die geschlitzte Bauweise des Halselements erleichtert.

Das Halselement kann weiterhin ein Rastelement umfassen, um eine feste Verbindung mit dem Kopfelement und/oder dem Stammelement zu ermöglichen. Es können auch mehrere solche Rastelemente an dem Halselement vorgesehen sein, beispielsweise ein Rastelement zur Verbindung mit dem Kopfelement und ein Rastelement zur Verbindung mit dem Stammelement. In einer Ausführungsform weist das Halselement zwei ringförmige Stege auf, und das Halselement und das Stammelement weisen jeweils Rastelemente auf, die jeweils in diese Stege eingreifen. Die Rastelemente können als Schnapphaken, Spundung oder andere formschlüssige Verbindung ausgestaltet sein.

Das Halselement ist ausgebildet und eingerichtet, mit einem vormontierten Kopfelement und/oder einem vormontierten Stammelement zu einem vollständig zusammengefügten Zustand der Gießform zusammengesetzt zu werden. Ein vormontiertes Kopfelement ist ein Kopfelement, bei dem die Oberschale mit der Unterschale fest verbunden ist, beispielsweise mithilfe eines Rastelements. Ein vormontiertes Stammelement ist ein Stammelement, bei dem die Oberschale mit der Unterschale fest verbunden ist, beispielsweise mithilfe eines Rastelements. Auf diese Weise kann das Kopfelement bzw. Stammelement mit Knochenzementteig befüllt werden, bevor es mit dem Halselement verbunden wird, um die Gießform komplett befüllt zusammenzusetzen. Wenn das Kopfelement und das Stammelement getrennt voneinander befüllbar sind, d.h. wenn die Gießform in einem noch nicht vollständig zusammengesetzten Zustand befüllbar ist, kann die Gießform leichter vollständig mit Knochenzementteig befüllt werden, insbesondere bei der Verwendung von hochviskosem Knochenzementteig. Dies vermeidet beim Befüllen mit Knochenzementteig die Bildung von Luftblasen innerhalb der Gießform, und erleichtert somit die Herstellung eines defektfreien Spacers.

In einer weiteren Ausführungsform weist das Stammelement ein Hebelelement auf. Das Hebelelement kann angeordnet und eingerichtet sein, die Oberschale und die Unterschale des Stammelements auseinander zu bewegen, um ein am Stammelement angeordnetes Rastelement und/oder Verbindungselement zu lösen. Vorteilhafterweise kann das Hebelelement am distalen Ende des Stammelements angeordnet sein, d. h. an demjenigen Ende des Stammelements, welches dem Kopfelement gegenüberliegt.

Die Oberschale und/oder Unterschale des Kopfelements ist mehrteilig ausgebildet. Beispielsweise kann die Unterschale des Kopfelements aus zwei symmetrischen Teilen zusammengesetzt sein. Die Teile können über eine formschlüssige Verbindung miteinander verbindbar sein, beispielsweise eine Spundung.

Alternativ oder zusätzlich kann in einer Ausführungsform das Kopfelement ein Befestigungselement umfassen, welches eingerichtet ist, die mehrteilig ausgebildete Oberschale und/oder Unterschale des Kopfelements zusammenzuhalten. Beispielsweise kann das Kopfelement eine Lasche umfassen, welche als Befestigungselement zwei parallele Stege aufweist, die jeweils in eine zu diesen Stegen komplementäre Struktur auf den beiden Teilen der Oberschale bzw. Unterschale eingreifen, um diese zusammenzuhalten.

Ein weiterer Aspekt der Erfindung betrifft ein Kit zur Herstellung eines Spacers, beispielsweise eines Hüftgelenkspacers, welches eine hierin beschriebene Gießform und einen Metallkern enthält, der eingerichtet ist, in das Stammelement eingebracht zu werden.

In einer Ausführungsform weist der Metallkern mehrere Flügelelemente auf, um den Metallkern innerhalb des Stammelements mittig auszurichten. Die Flügelelemente sind eingerichtet, einen Abstand zwischen dem Metallkern und der Wandung des Stammelements zu definieren.

In einer Ausführungsform weist das Kit mehrere Kopfelemente und/oder Stammelemente in jeweils unterschiedlicher Größe aufweist. Alternativ oder zusätzlich können auch Halselemente in unterschiedlicher Größe in dem Kit enthalten sein. In einigen Ausführungsformen sind die Kopfelemente, Stammelement und/oder Halselemente untereinander beliebig kombinierbar. Hierdurch können unterschiedlich konfigurierte Spacer hergestellt werden, welche eine bessere Passgenauigkeit für unterschiedliche Patienten bieten können.

Das Kit kann weiterhin einen Metallkern zur Einbringung in das Stammelement umfassen. Das Kit kann weiterhin Ausgangsstoffe zur Herstellung eines Knochenzementteigs, beispielsweise PMMA-Knochenzementteig, umfassen.

Das Kit kann weiterhin ein Antibiotikum umfassen, welches zur Einbringung in den Knochenzementteig vorgesehen ist.

Ein weiterer Aspekt der Erfindung betrifft ein Kit, welches eine Gießform mit einem Kopfelement, einem Halselement und einem Stammelement enthält, wobei das Kit mehrere Kopfelemente, Stammelemente und Halselement in jeweils unterschiedlicher Größe aufweist. Die Kopfelemente, Stammelement und Halselemente sind bevorzugt untereinander unabhängig von ihrer Größe beliebig kombinierbar. Das Kit kann weiterhin einen Metallkern zur Einbringung in das Stammelement umfassen. Das Kit kann weiterhin Ausgangsstoffe zur Herstellung eines Knochenzementteigs, beispielsweise PMMA-Knochenzementteig, umfassen.

Das Kit kann weiterhin ein Antibiotikum umfassen, welches zur Einbringung in den Knochenzementteig vorgesehen ist.

Die erfindungsgemäße Gießform kann mit Knochenzementteig befüllt werden. Mit "Knochenzementteig" wird hierin eine formbare Masse bezeichnet, die zu einem Knochenzement aushärtbar ist. Handelsübliche Knochenzemente werden in vielen Fällen als Kit angeboten, welches eine flüssige und eine feste Komponente enthält. Durch Mischen der flüssigen mit der festen Komponente kann der Anwender einen Knochenzementteig herstellen, der für eine begrenzte Zeit leicht verformbar ist. Bereits kurze Zeit nach dem Mischen wird der Knochenzementteig klebfrei, d. h. er haftet bei leichter Berührung mit einem Handschuh nicht mehr an diesem an (wie definiert in ISO 5833:2002). In diesem Zustand kann ein Knochenzementteig bevorzugt in ein Formnest der erfindungsgemäßen Vorrichtung zur Herstellung von Spacern eingefüllt werden, beispielsweise mithilfe eines Rührspatels oder einer geeigneten Austragsvorrichtung. Handelsübliche Knochenzemente werden häufig auf Basis von Polymethylmethacrylat (PMMA) angeboten. Die erfindungsgemäße Gießform kann bevorzugt mit einem solchen PMMA-Knochenzement verwendet werden. Es ist jedoch grundsätzlich auch möglich, andere Arten von Knochenzementen zu verwenden.

In einigen Ausführungsformen ist die Gießform insbesondere auch für die Befüllung mit hochviskosem Knochenzementteig geeignet. Hierzu ist die hierin beschriebene mehrteilige Bauweise vorteilhaft, wobei die Gießform ein Kopfelement und ein Stammelement aufweist, wobei das Kopfelement und das Stammelement jeweils unabhängig voneinander getrennt mit Knochenzementteig befüllt werden können. Bevorzugt sind das Kopfelement und/oder das Stammelement eingerichtet, in einem Zustand mit Knochenzementteig befüllt zu werden, in welchem die Oberschale und die Unterschale des Kopfelements und/oder des Stammelements bereits miteinander verbunden sind, und dadurch einen Hohlraum ausbilden.

Das Kopfelement und das Stammelement sind über ein Halselement miteinander verbindbar, sodass eine Gießform gebildet wird, welche die Herstellung eines Spacers in einem einzigen Gießvorgang erlaubt, sodass das Kopfteil und der Schaft des Spacers nicht getrennt voneinander hergestellt und nachfolgend miteinander verbunden werden müssen.

Die Gießform kann derart ausgestaltet sein, dass ein Spacer ohne Anguss herstellbar ist. Dies bedeutet, dass nach Aushärten des Knochenzements in der Gießform unmittelbar die gewünschte Geometrie des Spacers hergestellt werden kann, ohne dass Teile des ausgehärteten Knochenzements mechanisch abgetragen werden müssen. Auch ohne das Vorhandensein eines Anguss kann es jedoch vorteilhaft sein, den hergestellten Spacer zu entgraten. Dementsprechend werden geringfügige Grate, die beim Gießen des Spacers beispielsweise an denjenigen Stellen entstehen können, an denen verschiedene Teile der Gießform, zum Beispiel eine Oberschale und eine Unterschale, miteinander verbunden sind, hierin nicht als "Anguss" bezeichnet. Kleinere Strukturen, welche durch Austritt von Knochenzementteig an den Entlüftungsöffnungen entstehen können, werden hierin ebenfalls nicht als "Anguss" verstanden. Zur Bereitstellung einer Gießform, die die Herstellung eines Spacers ohne Anguss erlaubt, kann die hierin beschriebene modulare Bauweise der Gießform vorteilhaft sein. Das Kopfelement und das Stammelement können getrennt voneinander mit Knochenzementteig befüllt werden, und danach miteinander verbunden werden, gegebenenfalls über ein dazwischenliegendes Halselement.

Die erfindungsgemäße Gießform kann grundsätzlich aus jedem geeigneten Material bestehen. Biegsame, elastische Materialien können die Entnahme der abgeformten Spacer erleichtern. Die Gießform kann ein Metall und/oder Polymer umfassen. Beispiele für geeignete Polymere umfassen Kautschuk, Silikonkautschuk, Synthesekautschuk, Ethylen-Propylen-Dien-Kautschuk (EPDM), Polyethylen, Polyetheretherketon, und Polypropylen. Vorrichtungen aus Polypropylen erlauben eine besonders leichte Entnahme des ausgehärteten Knochenzements aus der Vorrichtung. In einigen Ausführungsformen ist die Vorrichtung aus einem transparenten oder zumindest transluzenten, d. h. optisch durchscheinenden, Material gebildet. Hierbei bieten sich entsprechende transparente oder transluzente Polymere an. Hierdurch kann der Verwender der Vorrichtung kontrollieren, ob die Gießform vollständig mit Knochenzementteig gefüllt ist.

Weiterhin kann die Gießform derart ausgestaltet sein, dass sie die Herstellung von Spacern mit unterschiedlicher Halslänge erlaubt, ohne dass der Anwender mithilfe eines Schneidwerkzeugs Anpassungen an der Gießform oder dem abgeformten Spacer vornehmen muss. Dies kann beispielsweise dadurch ermöglicht werden, dass die hierin beschriebenen Kopfelemente und Stammelemente jeweils mit Halselementen unterschiedlicher Größe verbindbar sind.

Ein weiterer Aspekt betrifft einen Spacer, der mit einer hierin beschriebenen Gießform herstellbar ist. Bevorzugt ist der Spacer zur Verwendung im Bereich einer Hüfte, insbesondere eines Hüftgelenks, vorgesehen. Bevorzugt handelt es sich um einen Hüftgelenkspacer. Ein solcher Hüftgelenkspacer ist bevorzugt mithilfe einer hierin beschriebenen Vorrichtung herstellbar. In einer Ausführungsform ist der Spacer ein Schultergelenkspacer.

In einer Ausführungsform umfasst der Spacer Knochenzement, oder besteht daraus. Bevorzugt umfasst der Knochenzement PMMA. Der Knochenzement kann ein Antibiotikum umfassen. Beispiele für geeignete Antibiotika umfassen Gentamycin, Clindamycin und Vancomycin. Der Spacer ist bevorzugt eingerichtet, im Zusammenhang mit einem chirurgischen Eingriff ein natürliches Gelenk, beispielsweise ein Hüftgelenk, eines Patienten temporär zu ersetzen.

Die Oberschale und die Unterschale des Kopfelements und/oder des Stammelements können grundsätzlich durch jede geeignete formschlüssige Verbindungen miteinander verbindbar sein, beispielsweise eine Spundung nach dem Nut-/Feder-Verbindungsprinzip. Es können hierzu jeweils mehrere gleichartige oder verschiedenartige Verbindungen vorgesehen sein.

Ein weiterer Aspekt, welcher nicht Teil der vorliegenden Erfindung ist, betrifft ein Verfahren zur Herstellung eines Spacers, bevorzugt eines Hüftgelenkspacers, umfassend die nachfolgenden Schritte,
(i) Bereitstellen einer Gießform, welche ein Kopfelement, welches eine Oberschale und eine Unterschale aufweist, ein Stammelement, welches eine Oberschale und eine Unterschale aufweist, und ein Rastelement aufweist,
(iia) gegebenenfalls Verbinden der Oberschale des Kopfelements mit der Unterschale des Kopfelements mithilfe des Rastelements, und/oder
(iib) gegebenenfalls Verbinden der Oberschale des Stammelements mit der Unterschale des Stammelements;
(iii) Einbringen eines Knochenzementteigs in das Kopfelement und das Stammelement,
(iv) Verbinden des Kopfelements und des Stammelements zu einer geschlossenen Gießform;
(v) Aushärten des Knochenzementteigs,
(vi) Öffnen des Kopfelements und/oder des Stammelements durch Lösen des Rastelements, und
(vii) dadurch Erhalten eines Spacers, bevorzugt eines Hüftgelenkspacers, aus Knochenzement.

In einem ersten Schritt des Verfahrens (i) wird eine Gießform bereitgestellt, welche ein Kopfelement, welches eine Oberschale und eine Unterschale aufweist, ein Stammelement, welches eine Oberschale und eine Unterschale aufweist, und ein Rastelement aufweist. Verschiedene Beispiel einer solchen Gießform sind hierin andernorts beschrieben.

In einem zweiten Schritt des Verfahrens (iia) werden gegebenenfalls die Oberschale und Unterschale des Kopfelements miteinander verbunden, falls sie noch nicht entsprechend vormontiert vorliegen. Hierzu können, wie hierin andernorts ausführlicher beschrieben, gegebenenfalls vorgesehene Verbindungselemente genutzt werden.

In entsprechender Weise werden gegebenenfalls in einem Schritt des Verfahrens (iib) die Oberschale und Unterschale des Stammelements miteinander verbunden, falls sie noch nicht entsprechend vormontiert vorliegen. Hierzu können, wie hierin andernorts ausführlicher beschrieben, gegebenenfalls vorgesehene Verbindungselemente genutzt werden.

In einem dritten Schritt des Verfahrens (iii) wird Knochenzementteig in das Kopfelement und das Stammelement eingebracht, zum Beispiel mit einer geeigneten Knochenzementteig-Austragsvorrichtung, oder von Hand.

In einem vierten Schritt des Verfahrens (iv) werden das Kopfelement und das Stammelement zu einer geschlossenen Gießform zusammengesetzt. Gegebenenfalls kann dazu ein Halselement verwendet werden, welches das Kopfelement mit dem Stammelement verbindet. Hierzu können beispielsweise die hierin andernorts ausführlicher beschriebenen Verbindungselemente eingesetzt werden.

In einem fünften Schritt (v) das Verfahrens wird der Knochenzementteig ausgehärtet. Der Knochenzementteig enthält typischerweise reaktive Bestandteile, die sich nach dem Anmischen des Knochenzementteigs innerhalb weniger Minuten durch chemische Reaktion miteinander verbinden, sodass ein hartes, nicht mehr verformbares Material gebildet wird. Dieses Material wird als (ausgehärteter) Knochenzement bezeichnet, und bildet den Spacer.

In einem sechsten Schritt (vi) wird das Kopfelement und/oder das Stammelement, in welchem sich der ausgehärtete Knochenzement befindet, geöffnet, indem das Rastelement gelöst wird. Hierdurch werden Oberschale und Unterschale des Kopfelements bzw. Stammelements voneinander getrennt, und geben den abgeformten Spacer frei.

Das Verfahren kann gegebenenfalls einen weiteren Schritt umfassen, in welchem ein Metallkern in das Stammelement eingebracht wird.

In einigen Ausführungsformen kann die Gießform ausgestaltet und eingerichtet sein, dasselbe Kopfelement und dasselbe Stammelement mit unterschiedlich großen Halselementen zu verbinden, wie hierin andernorts ausführlicher beschrieben.

Das oben beschriebene Verfahren kann vollständig außerhalb des menschlichen Körpers durchgeführt werden, d. h. *ex vivo.*

In einer Ausführungsform erfolgt die Durchführung des Verfahrens in der oben dargestellten Reihenfolge (i) bis (vii).

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

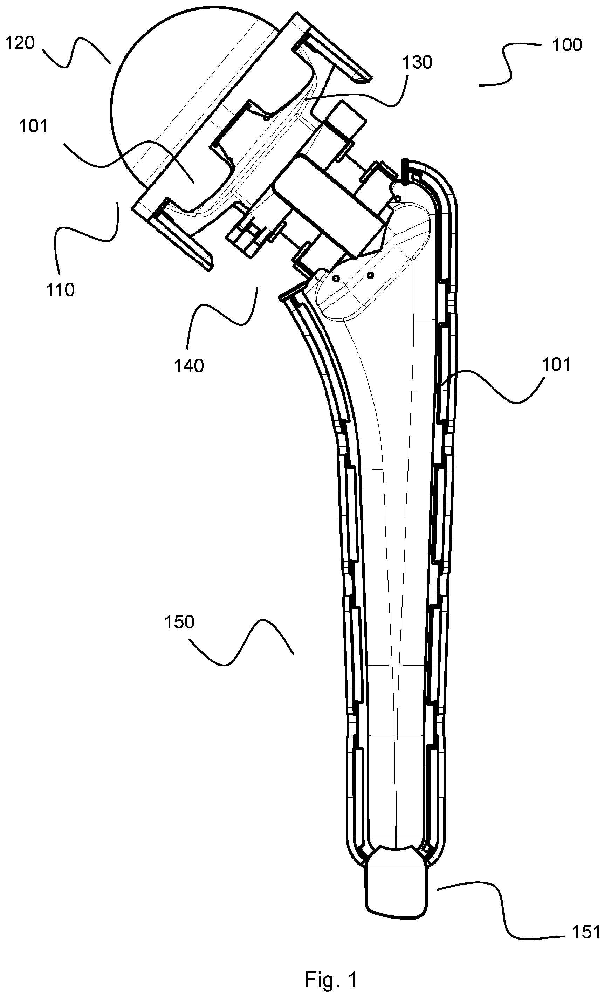

**Figur 1** zeigt beispielhaft eine Ausführungsform der Erfindung. Eine Gießform **100** weist ein Kopfelement **110** auf, welches aus einer Oberschale **120** und einer Unterschale **130** gebildet ist. Die Oberschale **120** und die Unterschale **130** werden mithilfe eines Rastelements **101** zusammengehalten. In dem in Figur 1 gezeigten Beispiel sind sechs Rastelemente in Form von Laschen an der Oberschale **120** angebracht, welche jeweils als Schnapphaken ausgebildet sind. Die Gießform **100** weist weiterhin ein Stammelement **150** auf, welches ähnlich wie das Kopfelement aus einer Oberschale und einer Unterschale gebildet ist. Ähnlich wie das Kopfelement weist auch das Stammelement **150** Rastelemente **101** auf, welche als Laschen mit Schnapphaken ausgebildet sind, und die Oberschale und die Unterschale des Stammelements **150** formschlüssig zusammenhalten. Am distalen Teil des Stammelements **150** ist weiterhin ein Hebelelement **151** angeordnet, welches eine Demontage des Stammelements erlaubt, indem über das Hebelelement **151** die Oberschale und die Unterschale des Stammelements auseinandergedrückt werden. Dies ermöglicht eine einfachere Entnahme des mithilfe der Gießform hergestellten Spacers.
   Zwischen dem Kopfelement **120** und dem Stammelement **150** ist ein Halselement **140** angeordnet, welches das Kopfelement **120** mit dem Stammelement **150** verbindet.

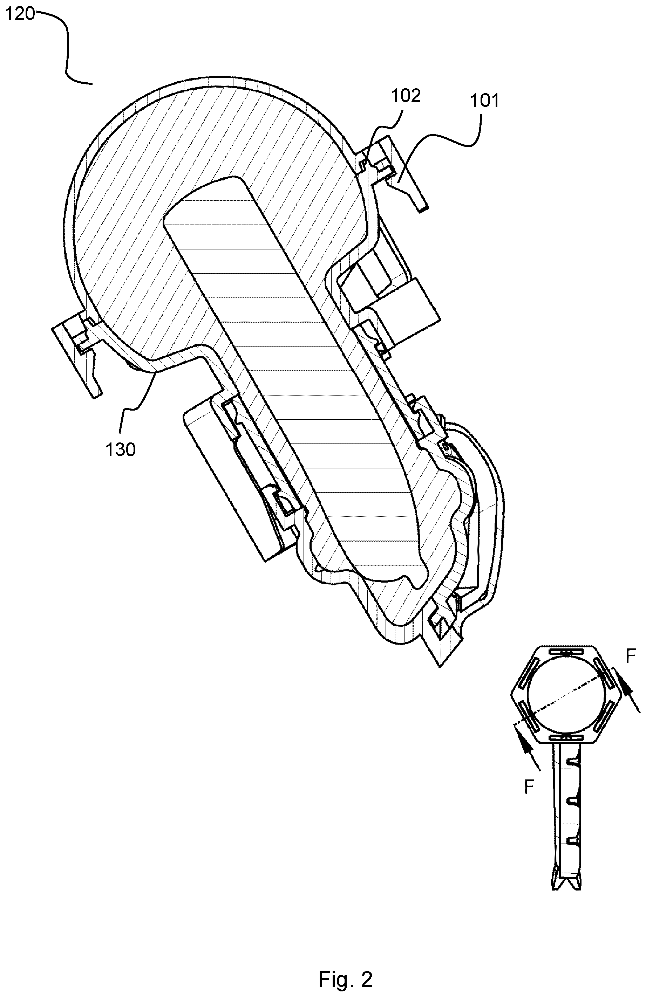

**Figur 2** zeigt einen Querschnitt durch eine erfindungsgemäße Gießform im Bereich des Kopfelements. Die Oberschale **120** weist ein Rastelement **101** auf, welches hier als laschenförmiger Schnapphaken ausgebildet ist. Das Rastelement hält Oberschale **120** und Unterschale **130** mithilfe einer formschlüssigen Verbindung zusammen. Zusätzlich zu dem Rastelement **101** ist die Oberschale **120** mit der Unterschale **130** über ein Verbindungselement **102** verbunden, welches hier als ein Paar ineinandergreifender Stege ausgestaltet ist, die jeweils auf Oberschale **120** und Unterschale **130** angeordnet sind.

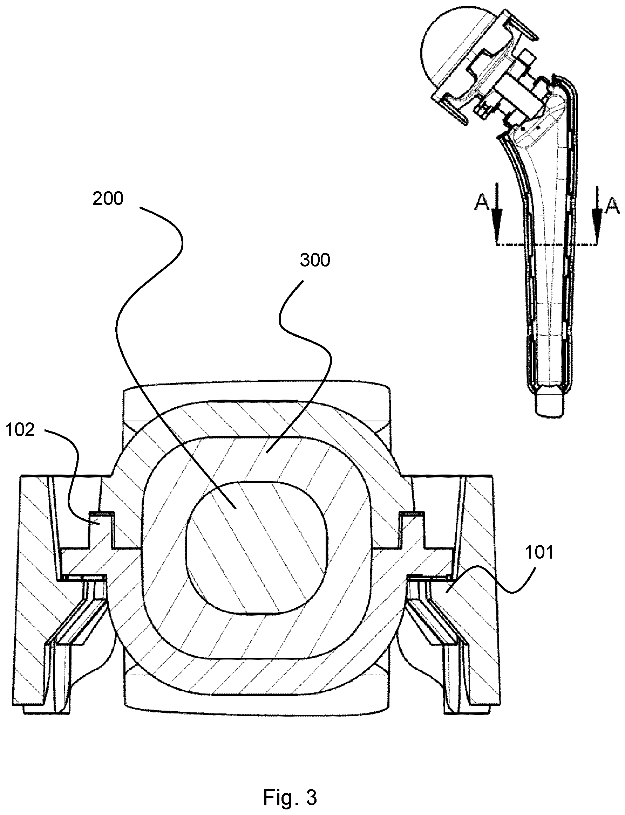

**Figur 3** zeigt einen Querschnitt durch eine erfindungsgemäße Gießform im Bereich des Stammelements. Ähnlich wie in Figur 2 für das Kopfelement gezeigt, weist auch das Stammelement ein Rastelement **101** auf, welches ebenfalls als laschenförmiger Schnapphaken ausgebildet ist. Es können mehrere solcher Rastelemente auf beiden Seiten des Stammelements vorgesehen sein, beispielsweise am umlaufenden Rand der Oberschale oder der Unterschale des Stammelements. Zudem sind, ähnlich wie in Figur 2 für das Kopfelement gezeigt, auch beim Stammelement Verbindungselemente **102** vorhanden, welche unabhängig vom Rastelement **101** die Oberschale mit der Unterschale des Stammelements verbinden. Im hier gezeigten Beispiel handelt es sich bei den Verbindungselementen **102** um ineinandergreifender Stege auf der Oberschale und der Unterschale des Stammelements.

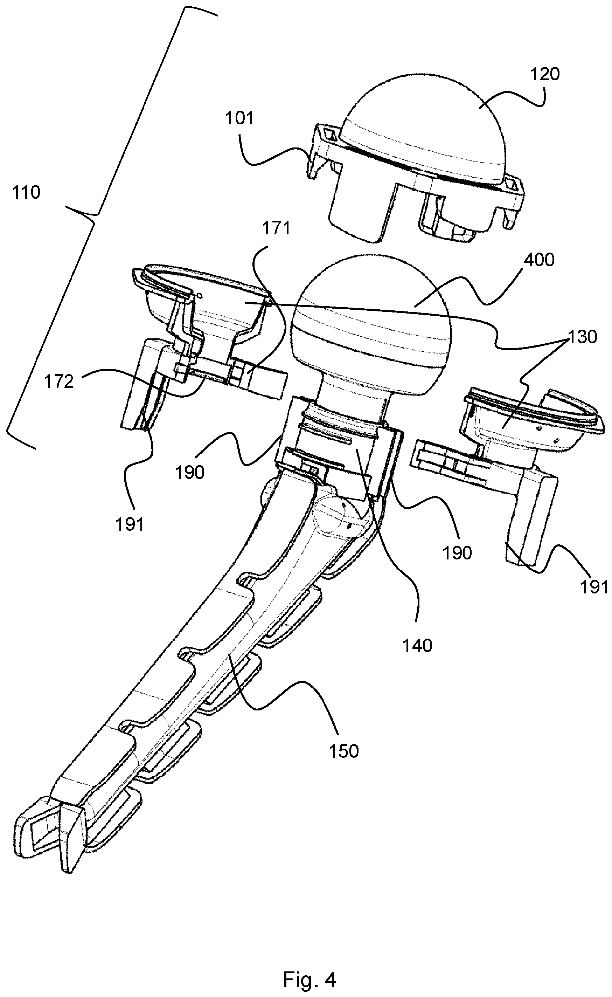

**Figur 4** zeigt eine Explosionszeichnung einer erfindungsgemäßen Gießform, und einen darin angeordneten Gelenkspacer **400,** welcher mithilfe der Gießform herstellbar ist. An der Oberschale **120** des Kopfelements **110** ist ein Schnapphaken als Rastelement **101** angeordnet. Im hier gezeigten Beispiel ist die Unterschale **130** des Kopfelements **110** zweiteilig ausgebildet, wobei die beiden Teile der Unterschale **130** mithilfe eines weiteren Rastelements **171,** welches als Schnapphaken ausgestaltet ist, und zusätzlich mithilfe eines weiteren Verbindungselements **172,** welches in diesem Beispiel als Spundung nach dem Nut/Feder-Verbindungsprinzip ausgeführt ist, miteinander verbunden werden können.
   Weiterhin ist ein Halselement **140** vorgesehen, welches jeweils formschlüssig mit dem Kopfelement **110** und dem Stammelement **150** verbunden ist. Das Halselement **140** hat hier die Form eines geschlitzten Rings, und weist zwei parallel angeordnete Stege **190** auf, welche jeweils parallel zur Ringachse des Halselements **140** angeordnet sind. Im hier gezeigten Beispiel weist das Ringelement **140** zwei Paare solcher Stege auf, die gegenüberliegend auf der Außenseite des Ringelements angeordnet sind. Die beiden Teile der Unterschale **130** des Kopfelements **110** weisen jeweils zu diesen Stegen komplementäre Strukturen **191** auf, welche eine formschlüssige Verbindung des Kopfelements **110** mit dem Ringelement **140** ermöglichen. Im hier gezeigten Beispiel können die Stegpaare **190** dazu in die Strukturen **191** eingeschoben werden, ähnlich wie es beispielsweise von Kabelschuhen bekannt ist.

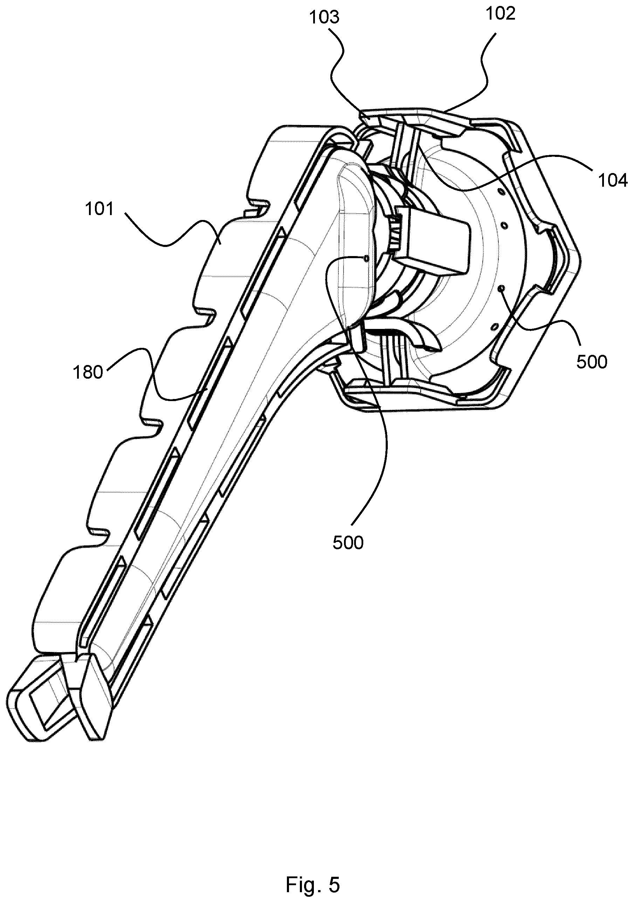

**Figur 5** zeigt eine isometrische Ansicht einer erfindungsgemäßen Gießform. Das Kopfelement **110** weist mehrere als Laschen ausgestaltete Rastelemente **101** auf, welche die Oberschale **120** mit der Unterschale **130** des Kopfelements mit Hilfe eines Schnapphakens verbinden. Zusätzlich sind an einigen der Laschen weitere Verbindungselemente **103** angebracht, die in komplementäre Strukturen **104** eingreifen, welche an der Unterschale **130** des Kopfelements angebracht sind. Die Verbindungselemente **103** verbinden die Oberschale **120** mit der Unterschale **130** des Kopfelements. Die Verbindung der Verbindungselemente **103** mit den komplementären Strukturen **104** ist in diesem Beispiel als Spundung nach dem Nut/Feder-Verbindungsprinzip ausgeführt, welche hier die seitliche Verdrehung der Oberschale **120** gegenüber der Unterschale **130** verhindert.
   Das Kopfelement weist weiterhin Öffnungen **500** auf, welche der Entlüftung dienen, wenn die Gießform mit Knochenzementteig befüllt wird. Solche Öffnungen können auch am Stammelement **150** vorgesehen sein.
   Das Stammelement **150** weist als Laschen ausgestaltete Rastelemente **101** auf, welche einen Hohlraum **180** zwischen diesen Laschen und der Wandung des Stammelements **150** definieren. Dieser Hohlraum **180** kann gegebenenfalls zwischen der Oberschale **160** und der Unterschale **170** austretenden Knochenzementteig aufnehmen, ohne dass dieser auf die Hand des Verwenders der Gießform gelangt.

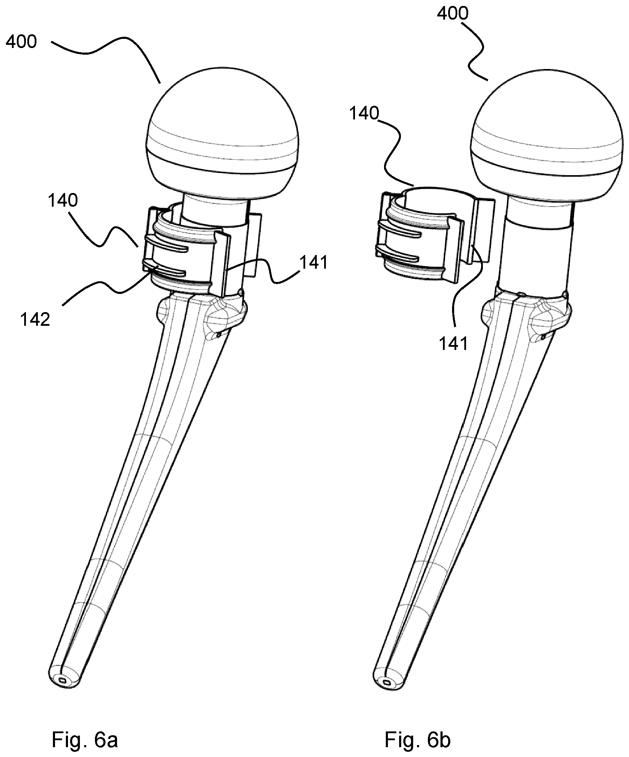

**Figur 6a** zeigt einen mithilfe einer hierin beschriebenen Gießform herstellbaren Spacer, nachdem das Kopfelement und das Stammelement entfernt worden sind. Das Halselement **140** ist noch am Spacer **400** verblieben. Das Halselement **140** weist einen Schlitz **141** auf, welcher die Wandung des Halselements unterbricht. Dies ermöglicht eine Verformung des Halselements **140.** Weiterhin weist das Halselement **140** einen Anschlag **142** auf, der seitlich umlaufend und orthogonal zur Ringachse des Halselements **140** angeordnet ist. Der Anschlag **142** ist angeordnet und eingerichtet, beim Zusammenbau der Gießform einen gewünschten Abstand zwischen Halselement **140** und Kopfelement **110** bzw. zwischen Halselement **140** und Stammelement **150** zu definieren.
**Figur 6b** zeigt, wie durch Auseinanderbiegen des Halselements **140** dieses vom Spacer **400** entfernt werden kann.

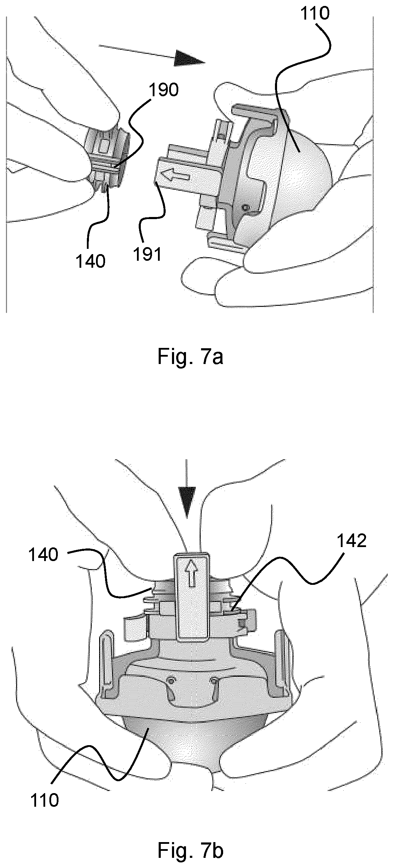

**Figur 7a** zeigt einen Schritt des Zusammenbaus einer erfindungsgemäßen Gießform. Hierbei wird ein Halselement **140** in ein Kopfelement **110** eingeschoben, wobei die Oberschale **120** und die Unterschale **130** des Kopfelements bereits verbunden sind. Hierbei werden am Halselement angeordnete Stege **190** in komplementäre Strukturen **191** eingeschoben, die am Kopfelement **110** angeordnet sind.
**Figur 7b** zeigt einen Schritt des Zusammenbaus einer erfindungsgemäßen Gießform. Das Halselement weist einen Anschlag **142** auf. Das Halselement **140** wird in das Kopfelement **110** eingeschoben, wobei der Anschlag **142** die Tiefe des Einschubs begrenzt.

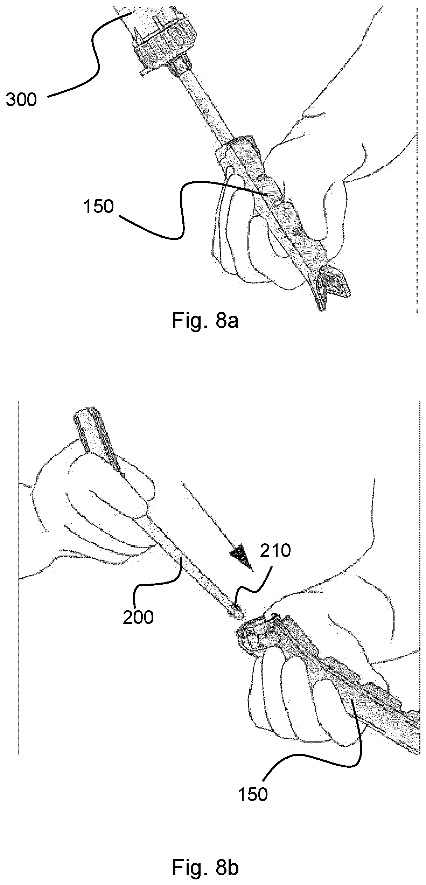

**Figur 8a** zeigt einen weiteren Schritt des Zusammenbaus einer erfindungsgemäßen Gießform. In das Stammelement **150,** dessen Oberschale und Unterschale miteinander verbunden sind, wird mithilfe einer geeigneten Austragsvorrichtung Knochenzementteig **300** eingebracht, bis dessen Innenraum vollständig mit Knochenzementteig befüllt ist.
**Figur 8b** zeigt einen weiteren Schritt des Zusammenbaus einer erfindungsgemäßen Gießform. Ein Metallkern **200,** welcher mehrere Flügelelemente **210** aufweist, wird in das bereits mit Knochenzementteig befüllte Stammelement **150** eingeführt. Die Flügelelemente **210** sind angeordnet und eingerichtet, eine zentrierte Anordnung des Metallkerns **200** innerhalb des Stammelements **150** zu ermöglichen.

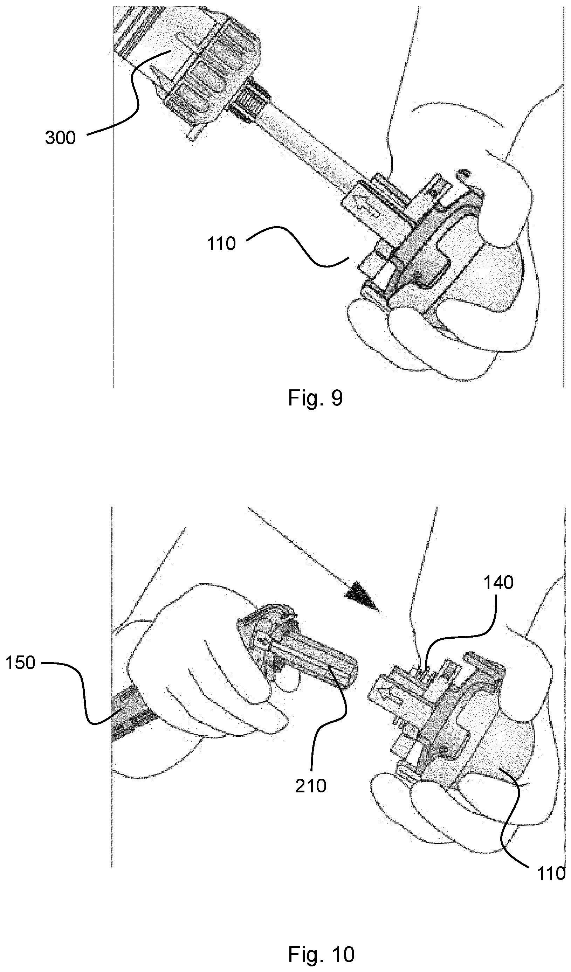

**Figur 9** zeigt einen weiteren Schritt des Zusammenbaus einer erfindungsgemäßen Gießform. Mithilfe einer Auftragsvorrichtung wird Knochenzementteig **300** in das Kopfelement eingebracht, während die Oberschale und die Unterschale des Kopfelements bereits miteinander verbunden sind, und das Kopfelement wie oben beschrieben mit dem Halselement **140** verbunden ist.
**Figur 10** zeigt einen weiteren Schritt des Zusammenbaus einer erfindungsgemäßen Gießform. Nach der Ausführung der oben beschriebenen Schritte wird das mit Knochenzementteig befüllte und mit einem Metallkern **210** versehene Stammelement **150** in das Halselement **140** eingeschoben, welches wiederum mit dem Kopfelement verbunden ist. Das Kopfelement ist ebenfalls bereits mit Knochenzementteig befüllt.

### LISTE DER BEZUGSZEICHEN

- 100: Gießform
- 101: Rastelement
- 102: Verbindungselement
- 103: Befestigungselement
- 104: zum Befestigungselement 103 komplementäre Struktur
- 110: Kopfelement
- 120: Oberschale des Kopfelements
- 130: Unterschale des Kopfelements
- 140: Halselement
- 141: Schlitz
- 142: Anschlag
- 150: Stammelement
- 151: Hebelelement
- 160: Oberschale des Stammelements
- 170: Unterschale des Stammelements
- 171: weiteres Rastelement
- 172: weiteres Verbindungselement
- 180: Hohlraum
- 190: Steg
- 191: zum Steg 190 komplementäre Struktur
- 200: Metallkern
- 210: Flügelelement
- 300: Knochenzementteig
- 400: Spacer
- 500: Öffnung

## Patentansprüche

1. Gießform zur Herstellung eines Gelenkspacers (100), aufweisend ein Kopfelement (110), welches eine Oberschale (120) und eine formschlüssig damit verbindbare Unterschale (130) aufweist, und ein Stammelement (150), welches eine Oberschale (160) und eine Unterschale (170) aufweist, wobei die Gießform ein Rastelement (101) aufweist, und eine Oberschale (120, 160) und eine Unterschale (130, 170) des Kopfelements (110) und/oder des Stammelements (150) mithilfe des Rastelements (101) miteinander verbindbar sind, **dadurch gekennzeichnet, dass** die Gießform ein Halselement (140) aufweist, welches mit dem Kopfelement (110) und dem Stammelement (150) verbindbar ist, und dass die Oberschale (120) und/oder die Unterschale (130) des Kopfelements (110) mehrteilig ausgebildet ist, und das Kopfelement ein Befestigungselement (103) umfasst, welches eingerichtet ist, die mehrteilig ausgebildete Oberschale (120) und/oder Unterschale (130) des Kopfelements (110) zusammenzuhalten, und das Halselement ausgebildet und eingerichtet ist, mit einem vormontierten Kopfelement und/oder einem vormontierten Stammelement zu einem vollständig zusammengefügten Zustand der Gießform zusammengesetzt zu werden.

2. Gießform nach Anspruch 1, weiterhin aufweisend ein formschlüssiges Verbindungselement (102), wobei eine Oberschale (120, 160) und eine Unterschale (130, 170) des Kopfelements (110) und/oder des Stammelements (150) mithilfe des formschlüssigen Verbindungselements (102) miteinander verbindbar sind.

3. Gießform nach einem der vorangehenden Ansprüche, wobei das Rastelement (101) als Lasche ausgebildet ist.

4. Gießform nach einem der vorangehenden Ansprüche, wobei das Kopfelement (110) und/oder das Stammelement (150) mehrere gleichartige Rastelemente (101) aufweist.

5. Gießform nach einem der vorangehenden Ansprüche, wobei das Rastelement angeordnet und eingerichtet ist, in einem vollständig zusammengefügten Zustand der Gießform einen Hohlraum (180) zur Aufnahme von überschüssigem Knochenzementteig zu definieren.

6. Gießform nach einem der vorangehenden Ansprüche, wobei das Halselement weiterhin einen Steg (190) aufweist, der parallel zur Mittelachse des Halselements angeordnet ist, wobei der Steg angeordnet und eingerichtet ist, in eine zu dem Steg komplementäre Struktur (191) einzugreifen, die an dem Kopfelement oder dem Stammelement angeordnet ist.

7. Gießform nach einem der vorangehenden Ansprüche, wobei das Halselement (140) einen Schlitz (141) aufweist, der parallel zur Mittelachse des Halselements (140) angeordnet und eingerichtet ist, ein Auseinanderbiegen oder Zusammendrücken des Halselements (140) zu ermöglichen.

8. Gießform nach einem der vorangehenden Ansprüche, wobei das Stammelement (150) ein Hebelelement (151) aufweist, das angeordnet und eingerichtet ist, die Oberschale (160) und die Unterschale (170) des Stammelements (150) auseinander zu bewegen, um ein am Stammelement angeordnetes Rastelement (101) und/oder Verbindungselement (102) zu lösen.

9. Gießform nach einem der vorangehenden Ansprüche, wobei das Rastelement (101) als Lasche ausgebildet und an dem Kopfelement (110) angeordnet ist, und wobei die Lasche (101) weiterhin ein Befestigungselement (103) umfasst, das angeordnet ist, in eine zu dem Befestigungselement (103) komplementäre Struktur (104) des Kopfelements (110) einzugreifen, um die mehrteilig ausgebildete Oberschale (160) und/oder Unterschale (130) des Kopfelements (110) zusammenzuhalten.

10. Kit zur Herstellung eines Hüftgelenkspacers, aufweisend eine Gießform gemäß einem der vorangehenden Ansprüche und einen Metallkern (200), der eingerichtet ist, in das Stammelement (150) eingebracht zu werden.

11. Kit nach Anspruch 10, wobei der Metallkern (200) mehrere Flügelelemente (210) aufweist, um den Metallkern innerhalb des Stammelements (150) mittig auszurichten.

12. Kit zur Herstellung eines Spacers, aufweisend eine Gießform gemäß einem der Ansprüche 1 bis 9, wobei das Kit mehrere Kopfelemente (120), Halselemente (140) und/oder Stammelemente (150) in jeweils unterschiedlicher Größe aufweist, welche bevorzugt untereinander beliebig kombinierbar sind.

## Claims

1. A casting mold for producing a joint spacer (100), comprising a head element (110), which comprises an upper shell (120) and a lower shell (130) which can be form-fittingly connected thereto, and a stem element (150), which comprises an upper shell (160) and a lower shell (170), wherein the casting mold comprises a latching element (101), and an upper shell (120, 160) and a lower shell (130, 170) of the head element (110) and/or of the stem element (150) can be connected to one another by means of the latching element (101), **characterized in that** the casting mold comprises a neck element (140) which can be connected to the head element (110) and the stem element (150), **and in that** the upper shell (120) and/or the lower shell (130) of the head element (110) is multipart, and the head element comprises a fastening element (103) which is designed to hold the multipart upper shell (120) and/or lower shell (130) of the head element (110) together, and the neck element is designed and configured to be combined with a pre-installed head element and/or a pre-installed stem element to form a fully assembled state of the casting mold.

2. The casting mold according to claim 1, further comprising a form-fitting connecting element (102), wherein an upper shell (120, 160) and a lower shell (130, 170) of the head element (110) and/or of the stem element (150) can be connected to one another by means of the form-fitting connecting element (102).

3. The casting mold according to either of the preceding claims, wherein the latching element (101) is designed as a tab.

4. The casting mold according to any of the preceding claims, wherein the head element (110) and/or the stem element (150) comprises a plurality of identical latching elements (101).

5. The casting mold according to any of the preceding claims, wherein the latching element is arranged and configured to define a cavity (180) for receiving excess bone cement dough in a fully assembled state of the casting mold.

6. The casting mold according to any of the preceding claims, wherein the neck element further comprises a protrusion (190) arranged parallel to the central axis of the neck element, wherein the protrusion is arranged and configured to engage in a structure (191) which is complementary to the protrusion and which is arranged on the head element or the stem element.

7. The casting mold according to any of the preceding claims, wherein the neck element (140) comprises a slot (141) arranged parallel to the central axis of the neck element (140) and configured to allow the neck element (140) to be bent apart or pressed together.

8. The casting mold according to any of the preceding claims, wherein the stem element (150) comprises a lever element (151) arranged and configured to move the upper shell (160) and the lower shell (170) of the stem element (150) apart in order to release a latching element (101) and/or connecting element (102) arranged on the stem element.

9. The casting mold according to any of the preceding claims, wherein the latching element (101) is designed as a tab and is arranged on the head element (110), and wherein the tab (101) further comprises a fastening element (103) arranged to engage in a structure (104) of the head element (110) that is complementary to the fastening element (103) in order to hold the multipart upper shell (160) and/or lower shell (130) of the head element (110) together.

10. A kit for producing a hip joint spacer, comprising a casting mold according to any of the preceding claims and a metal core (200) configured to be introduced into the stem element (150).

11. The kit according to claim 10, wherein the metal core (200) comprises a plurality of wing elements (210) in order to centrally align the metal core within the stem element (150).

12. A kit for producing a spacer, comprising a casting mold according to any of claims 1 to 9, wherein the kit comprises a plurality of head elements (120), neck elements (140) and/or stem elements (150) in different sizes, which can preferably be combined with one another as desired.

## Revendications

1. Moule pour la fabrication d'un espaceur d'articulation (100), présentant un élément formant tête (110) qui présente une coque supérieure (120) et une coque inférieure (130) pouvant être reliée à celle-ci par complémentarité de forme, et un élément formant tige (150) qui présente une coque supérieure (160) et une coque inférieure (170), dans lequel le moule présente un élément d'encliquetage (101) et une coque supérieure (120, 160) et une coque inférieure (130, 170) de l'élément formant tête (110) et/ou de l'élément formant tige (150) peuvent être reliées entre elles à l'aide de l'élément d'encliquetage (101), **caractérisé en ce que** le moule présente un élément formant col (140) pouvant être relié à l'élément formant tête (110) et à l'élément formant tige (150), **et en ce que** la coque supérieure (120) et/ou la coque inférieure (130) de l'élément formant tête (110) sont réalisées en plusieurs parties, et l'élément formant tête comprend un élément de fixation (103) qui est conçu pour maintenir ensemble la coque supérieure (120) et/ou la coque inférieure (130) de l'élément formant tête (110) réalisées en plusieurs parties, et l'élément formant col est réalisé et conçu pour être assemblé avec un élément formant tête prémonté et/ou un élément formant tige prémonté pour obtenir un état entièrement assemblé du moule.

2. Moule selon la revendication 1, présentant en outre un élément de liaison (102) à complémentarité de forme, dans lequel une coque supérieure (120, 160) et une coque inférieure (130, 170) de l'élément formant tête (110) et/ou de l'élément formant tige (150) peuvent être reliées entre elles à l'aide de l'élément de liaison (102) à complémentarité de forme.

3. Moule selon l'une des revendications précédentes, dans lequel l'élément d'encliquetage (101) est réalisé sous la forme d'une languette.

4. Moule selon l'une des revendications précédentes, dans lequel l'élément formant tête (110) et/ou l'élément formant tige (150) présentent plusieurs éléments d'encliquetage (101) de même type.

5. Moule selon l'une des revendications précédentes, dans lequel l'élément d'encliquetage est disposé et conçu pour définir, dans un état entièrement assemblé du moule, une cavité (180) permettant de recevoir l'excédent de pâte de ciment osseux.

6. Moule selon l'une des revendications précédentes, dans lequel l'élément formant col présente en outre une barrette (190) disposée parallèlement à l'axe central de l'élément formant col, dans lequel la barrette est disposée et conçue pour venir en prise dans une structure (191) complémentaire de la barrette et disposée sur l'élément formant tête ou l'élément formant tige.

7. Moule selon l'une des revendications précédentes, **caractérisé en ce que** l'élément formant col (140) présente une fente (141) disposée parallèlement à l'axe central de l'élément formant col (140) et conçue pour permettre l'écartement ou la compression de l'élément formant col (140).

8. Moule selon l'une des revendications précédentes, dans lequel l'élément formant tige (150) présente un élément formant levier (151) qui est disposé et conçu pour écarter la coque supérieure (160) et la coque inférieure (170) de l'élément formant tige (150) afin de libérer un élément d'encliquetage (101) et/ou un élément de liaison (102) disposés sur l'élément formant tige.

9. Moule selon l'une des revendications précédentes, dans lequel l'élément d'encliquetage (101) est réalisé sous la forme d'une languette et est disposé sur l'élément formant tête (110), et dans lequel la languette (101) comprend en outre un élément de fixation (103) qui est disposé pour venir en prise dans une structure (104) de l'élément formant tête (110), laquelle structure est complémentaire de l'élément de fixation (103), afin de maintenir ensemble la coque supérieure (160) et/ou la coque inférieure (130) de l'élément formant tête (110) réalisées en plusieurs parties.

10. Kit pour la fabrication d'un espaceur d'articulation de la hanche, présentant un moule selon l'une des revendications précédentes et un noyau métallique (200) qui est conçu pour être inséré dans l'élément formant tige (150).

11. Kit selon la revendication 10, dans lequel le noyau métallique (200) présente plusieurs éléments formant ailettes (210) pour centrer le noyau métallique à l'intérieur de l'élément formant tige (150).

12. Kit pour la fabrication d'un espaceur, présentant un moule selon l'une des revendications 1 à 9, dans lequel le kit présente plusieurs éléments formant tête (120), éléments formant col (140) et/ou éléments formant tige (150) de tailles respectivement différentes qui peuvent de préférence être combinés entre eux de manière aléatoire.
